# EUROPEAN PATENT APPLICATION

(11) **EP 4 681 631 A1**
(43) Date of publication of application: **21.01.2026**
(21) Application number: 24769593.5
(22) Date of filing: 14.03.2024
(51) Int. Cl.: A61B 5/1172, G06F 21/32, G06N 3/02

(54) **PROCESS AND SYSTEM FOR COLLECTING IMAGES AND IDENTIFYING CHILDREN AND NEWBORNS**

(30) Priority: 16.03.2023 BR 102023004966
(71) Applicant: Natosafe Tecnologia da Informação S/A, 80250-104 Curitiba/PR (BR)
(72) Inventor: FILIPAK, Marcelo, 80530-320 Curitiba/PR (BR); JUNIOR, Luiz Antonio Zanlorensi, 84045-430 Ponta Grossa/PR (BR); WASILEVSKI, Ildefonso, 81900-470 Curitiba/PR (BR); MAZZETTO, Muriel, 80240-020 Curitiba/PR (BR); SILVA, Evandro Celino Da, 83325-180 Pinhais/PR (BR)
(74) Representative: Torner, Juncosa I Associats, SL
(86) International application number: PCT/BR2024/050095
(87) International publication number: WO 2024/187260

(57) **Abstract**

The present invention patent relates to the technical field of biometric identification, and more specifically relates to a process and system for collecting images and identifying children and newborns that comprises a biometric reading device (1.1), a collecting system (1.2) and a storage and consulting system (1.3). The image collecting system (1.2) comprises a subsystem for collecting digital images, a component for collecting a set of fingerprints (2.5) compiled from individual images of each fingerprint, a component for performing further checks and conversions (2.6) on the set of fingerprint images, and for validating the quality of said images, and a component for entering biographical data information (2.7) into the validated set of images. The subsystem for collecting digital images comprises a component for processing the images from the biometric reading device (2.1), a component for configuring and filtering (2.2), normalizing and other conventional methods of processing images, a component for providing and displaying the images of the information to a user (2.3), and a component for real-time checks (2.4) of previously configured images. The storage and consulting system (1.3), which acts as a repository for storing, maintaining and consulting the images and information collected, comprises an interface for storing (3.1), an interface for consulting (3.2), an interface for viewing and management (3.3) via web services, and a web page component (3.4) for direct viewing and management of the system by authorized users.

## Description

The present patent of invention relates to the technical field of biometric identification, and more specifically relates to a process and system for collecting images and identifying children and newborns.

### BACKGROUND

Civil identification is the first step towards citizenship, as it establishes a person's status as a citizen and grants them rights and responsibilities within society. However, notary offices are currently the weakest link in the national identification system. This is because birth certificates do not guarantee uniqueness as they are not linked to their bearers.

According to UN data, human trafficking is the second most lucrative branch of organized crime after drug and arms trafficking, particularly the disappearance of children, teenagers, and babies soon after childbirth. Official surveys present maps of organ trafficking routes.

In several different regions of Brazil, illegal organ trafficking and people disguising themselves as social workers to target poor pregnant women and convince them to give their babies up as soon as they are born are proven facts. It is quite common for requests to be made for the identification of children who were with women suspected of not being their mothers, or whose mothers' names were different from those on their birth certificates.

Even though it is provided for in the Brazilian legislation through Law 8069/90 - The Statute of Children and Adolescents, the current neonatal identification system is outdated, making identification difficult and favoring crimes such as child and organ trafficking, the mixing-up of babies in maternity wards, and ideological falsehood, since there are no devices capable of adequately identifying children and creating a link able to connect the child to the information contained in the birth certificate or to the legal guardians.

The current mechanism for collecting fingerprints of newborn babies using ink and paper is ineffective and cannot be used for collecting palmprints and fingerprints, as newborn babies could put their ink-stained hands in their mouths. The quality of the collection is also not guaranteed, as it depends on human effort and attention to detail, both of which are often overlooked. In addition, images obtained using ink and paper in the collection do not have adequate resolution for viewing newborn fingerprints in a sufficiently detailed manner.

Papilloscopic images are more appealing than other biometrics because they are non-invasive and easy to apply and accept, besides having the significant advantage of immutability, which makes them ideal for future identification purposes. However, using this process to collect fingerprints from newborn babies is difficult because the ridges on their fingerprints are, on average, 2.5 to 3 times smaller than those on adults' fingerprints and are more susceptible to deformation.

Currently, commercially available biometric systems are almost entirely used for identifying adults. Despite all the advances in biometric technology, few identification approaches have been developed for identifying newborns. This means ignoring the approximately one hundred thirty-one million births that occur worldwide each year. The problem is exacerbated when we consider the global population of children aged zero to five, which is approximately five hundred million.

The use of scanners, such as biometric readers, has increased in recent years, particularly for applications related to user identification. These readers capture fingerprint images and are associated with computer processes that recognize the fingerprints and identify the individual to whom they belong. Examples of these readers can be found in ATMs, banks, electronic sign-in systems, and patient registration systems.

It should be noted that, for the fingerprint registration and identification systems to be efficient and dependable, the captured images must meet certain resolution and sharpness standards. For instance, the FBI stipulates that biometric readers certified for identification must have a minimum resolution of 500ppi for identification and 1000ppi for registration. While these values are sufficient to capture good images and solve the details of adult fingerprints, the same cannot be said for newborns, whose fingerprints are significantly smaller than those of adults.

In an extensive review of the literature conducted to provide an overview of the current state of biometric readers, more specifically on processes and systems for collecting images and identifying children and newborns in general, the object of the present invention, documents relevant to the prior art related to the specific object claimed in the present invention were not described, however, a brief description of a few documents from the literature and from patents is necessary for a complete understanding of the intended technical field.

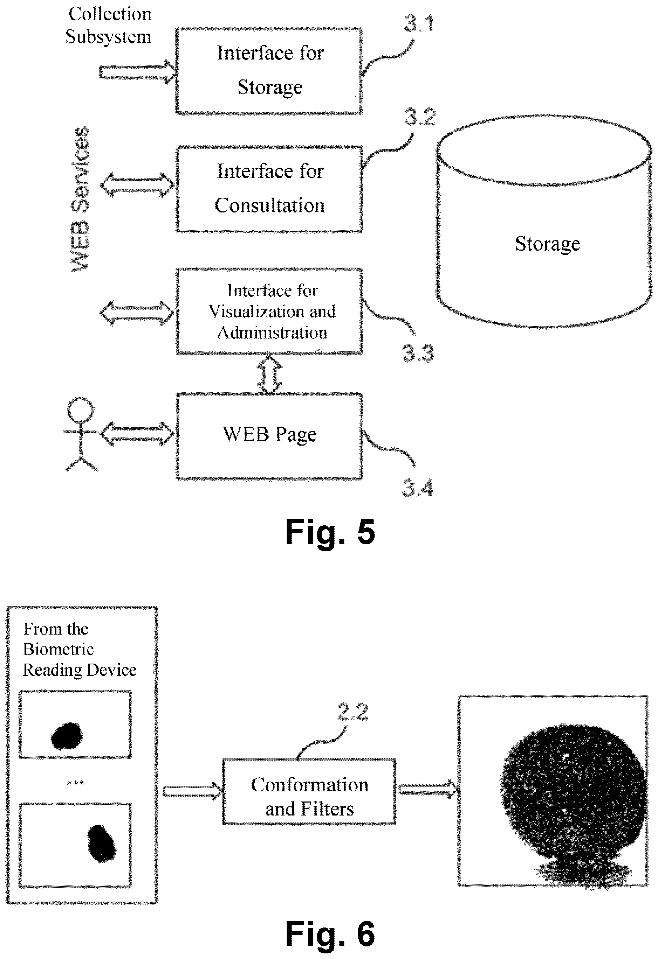

In the context of the non-patent literature, in the article by Koda, Higuchi, and Jain (Advances in Capturing Child Fingerprints: A High Resolution CMOS Image Sensor with SLDR Method, presented at BIOSIG 2016, p. 329), the authors describe a device capable of producing images with a resolution of 1270ppi and demonstrate that this device can distinguish the valleys of the crests from the fingerprints of a newborn, as illustrated in Figure 6 from the article.

In the context of patents, a large number of patent documents relate to biometric fingerprint readers, but most of these claim the image generation method (FTIR or scattering), the optical architecture, the use of special components to reduce distortion, and the use of films to improve image quality.

For example, in the context of fingerprint reading devices, patent document BR 102019016898-6 describes a high-resolution, portable biometric reader for newborns, while US patent document US2005169506 outlines a lightweight, portable device for capturing fingerprints.

### TECHNICAL ISSUES

In the context of non-patent literature, Figure 6 of the article by Koda, Higuchi, and Jain, as well as the figures in a later article by Jain et al (Fingerprint Recognition of Young Children, IEEE Transactions on Information Forensics and Security, Vol. 12, p. 1501, 2017), the image of the pores is consistent only from the third month of life, which makes obtaining a unique child identification difficult.

In turn, in the context of patent literature, none of the documents found deals with the application of a specific process and system for collecting images and identifying children and newborns to generate adequate images of babies' fingerprints, considering their requirements, portability, and usability.

In the specific case of patent document BR 102019016898-6, despite the fact that it describes a fingerprint reading device for newborns, it lacks an improved process and system for processing images of newborns. In turn, patent document US2005169506 presents a device that captures four or more adult fingerprints, with a capture surface area of more than 3x3 inches, which is still much larger than necessary for babies' fingerprints, and the resolution is not discussed.

Thus, the biometric identification of children and newborns is not widespread today, since the systems designed for adults are not suitable for this purpose.

A few of the inherent problems associated with child and newborn biometrics are:
- the size of the fingers compared to those of adults;
- rapid child growth, especially in the first years of life, causes substantial differences in size between collections of the same fingerprint from the same child;
- fingerprint morphology (especially in newborns) has larger pores and smaller spaces between papillae in relative comparison with adult individuals; and
- systems created for adults are not yet ready to treat children and newborn data uniformly.

Furthermore, in terms of the relevance of the conversions performed on the images, after overcoming the challenges of collecting an image with higher resolution (1500 or more) DPIs, it is observed that this is not enough for the extraction of minutiae by the currently available systems as these are developed and "calibrated" for the morphology of adult fingerprints.

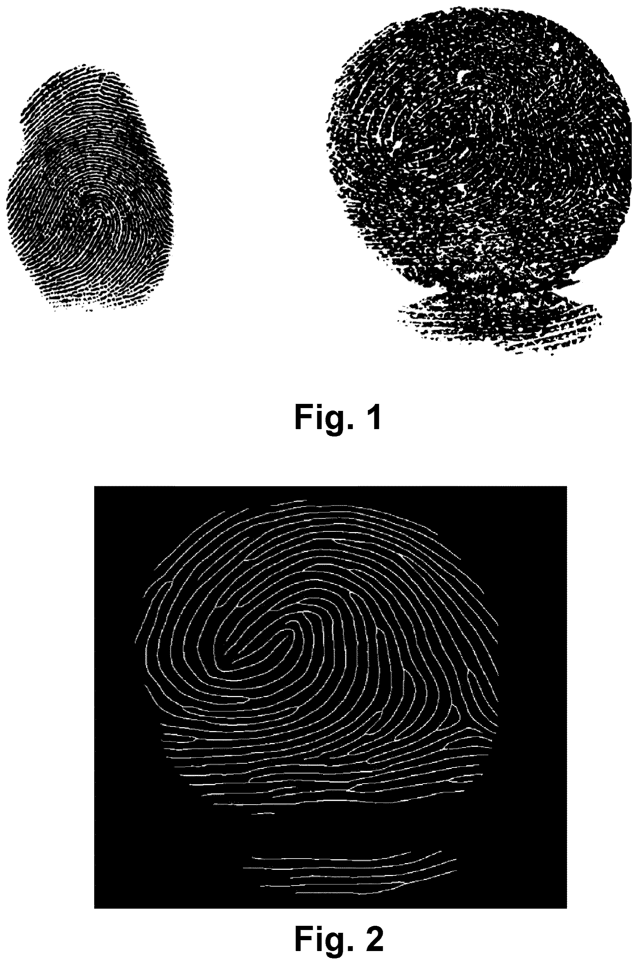

A direct attempt to place the images with scale corrections so that they are compatible with adult images is unsatisfactory. For example, as can be seen in Figure 1, when the image of the newborn (from the right) is resized to match the size (pixel) as the adult image, it is not recognized by biometric systems because the morphology is completely different.

Thus, while a few studies suggest that fingerprint images preserve their characteristics throughout an individual's growth, morphology (the relationship between the width of the valleys and ridges) is not preserved.

In the article published by Vanina Camacho et al (Recognizing Infants and Toddlers in the On-production Fingerprint Database, Conference: 2017 International Conference of the Biometrics Special Interest Group (BIOSIG)), a study is cited that acknowledges this fact, but does not detail how the image should be processed to maximize the capture of image minutiae, restricting itself to the segmentation for obtaining the total dimensions of the images.

### TECHNICAL SOLUTION

In terms of the necessary conversions to prepare the image obtained by the process and system for collecting images and identifying children and newborns, the present invention solves the problem faced in the state of the art. To make a newborn image compatible with the available minutiae extractors, conversions are carried out to clearly define the boundary between ridges and valleys. To achieve this, annotations are made by building a dataset with hundreds of images with varying capture qualities and the lines are annotated as shown in Figure 2.

This type of image has been tested using established matchers on the market (for instance, Bozorth and Verifinger) with very satisfactory results in terms of minutiae extraction. A dataset is then built by relating the base images (captured with the high-resolution scanner) to their annotation to train a neural network.

In the case of the model for automatic extraction of the valleys, several models are tested for image segmentation by using convolutional neural networks, for instance, the U-NET architecture (https://arxiv.org/abs/1505.04597) uses a MobileNet V2 (https://arxiv.org/abs/1704.04861) network with fast inference as a base.

However, initial studies show that processing the entire image would take the network a long time, as most reference models have an input format of around 224x224x3. Considering that high-resolution images of newborns are around 1200x1200 in size, a minimum total of 36 inferences would be required to process an image, making the process too time-consuming for real-time use. Furthermore, if there is an overlap between the frames to improve the quality of the final image, the number of inferences required would increase significantly (by at least fourfold).

Using appropriate hardware (with a larger memory), it is possible to train a model with an input image of 1280x1280.

Thus, the above-described model proves to be the most appropriate solution to the problem. Several other models tested either do not perform or simply do not converge in the case presented here (dense images of children's fingerprints with an input of 1280x1280 pixels). Therefore, the appropriate architecture for the process and system for collecting images and identifying children and newborns of the present invention is not trivial and could have been abandoned after some experimentation, due to poor performance or lack of convergence.

The first attempts to train the model using small datasets (around 10 images) did not converge; that is to say, they did not produce an output similar to that expected. To improve the quality of the markings, an even larger set of images was annotated again, and the results improved considerably. However, as this annotation process is very time-consuming and can take months or even years for a hundred images, the challenge of the present invention is to speed up this process.

Another approach used is to change the loss function for the network training so as to ignore the vicinity of the markings. This can be compared to considering markings as having three possible values: marked (valley), unmarked (not valley) and edge (adjacent to the valley where errors will be ignored). This technique significantly accelerates the initial training. Although it has the side effect of thickening the lines, this can easily be compensated for using traditional computer vision techniques.

When it comes to choosing and refining the dataset, once a trained model is available, it is possible to start manual markup with a markup suggestion (which is filled in by inference using the previously built model).

Candidate images for the model's dataset are selected by ordering a larger set of images according to their perceived quality, having a voting scheme where 2 images were presented on the screen and the specialist selected the one of better quality.

After that, the images can be spaced from lowest to highest quality, and a statistically significant sample of a few hundred images can be chosen in this way. In this set of images, initial markings are performed using the markings of the previously trained model as an 'accelerator'.

**In** addition to the larger dataset, data augmentation techniques are used to introduce real-time distortions to the images and labels during the training of the model.

Then, this cycle of training the model and refining the dataset is repeated at least 30 times until a result close to optimal is reached.

On the use of the model for image quality checking, in addition to extracting minutiae for registration or biometric authentication purposes, the valleys segmentation model can be used to check image quality and automatically detect fingerprint quality during capture.

The main requirement is to qualify an image immediately after capture (within 100ms). According to the present invention, this ability of the model allows images to be captured within optimal criteria, even if the person capturing them has no knowledge of image quality.

Regarding other characteristics of the images, the main class extracted from the images by the technique described here is the exact position of the fingerprint valleys. This technique can also be used to extract other characteristics, such as pores and dirt, and can be used to refine matching techniques and provide feedback to the user about actions during collection (e.g., wiping the child's finger).

More specifically, the process and system for collecting images and identifying children and newborns, as applied to the neonate biometric readers of the present invention, allows for the appropriate and satisfactory registration of babies' fingerprints. Consequently, this biometric identification system enables the generation of identification documents, such as birth certificates, civil identity records and passports, and controls physical access to maternity wards to help prevent the exchange or kidnapping of children and assist in the recovery and identification of babies and missing children.

The patent in question is characterized by the integration of system and process components in a differentiated design that meets the various requirements of the intended use, i.e., the acquisition of images of fingerprints, palmprints, and footprints of newborns (neonates). This design ensures that biometric reading scanners are highly efficient, functional, secure, versatile, precise and economical, thanks to their excellent added technical qualities, thus providing advantages and improvements in the routine acquisition of images of fingerprints, palmprints, and footprints of newborns, whose characteristics differ significantly from those widely known in the current state of the art.

### BRIEF DESCRIPTION OF THE DRAWINGS

The following description is not limited to the drawings or components cited but is referenced by the illustrations below.
Figure 1 shows scale-corrected comparative images of a newborn and an adult.
Figure 2 shows a converted image of a newborn compatible with available minutiae extractors.
Figure 3 shows a diagram with an overview of the process and system for collecting images and identifying children and newborns according to the present invention.
Figure 4 shows a diagram detailing the components of the collecting system according to the present invention.
Figure 5 shows a diagram detailing the components of the storage and consulting system according to the present invention.
Figure 6 shows a diagram detailing the configuring and filtering component according to the present invention.
Figure 7 shows a diagram detailing the component that carries out the real-time checks according to the present invention.
Figure 8 shows a diagram detailing the component that carries out further checks and conversions according to the present invention.
Figure 9 shows an image with indications of the distances between the valleys, which can be normalized to create an age-invariant template.

### DETAILED DESCRIPTION

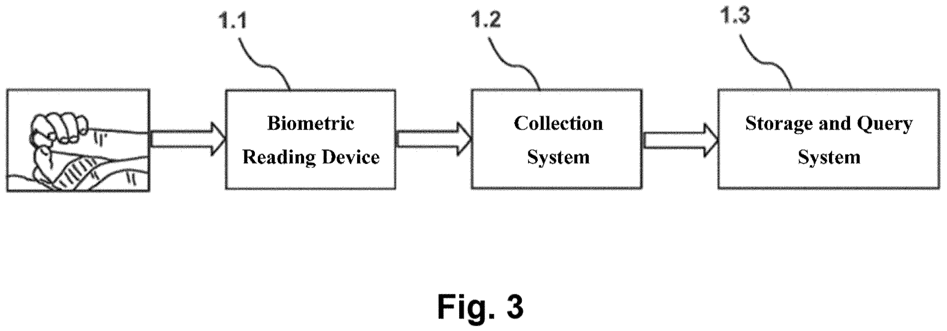

According to the present invention, the process and system for collecting images and identifying children and newborns comprises the parts illustrated in the diagram of Figure 3.

Figure 1 indicates the difference between the fingers of a newborn and those of an adult.

The biometric reading device (1.1) of the system, such as a scanner, must be able to focus on and collect images at a resolution higher than the current standard of 500 DPI for adults. The system of the present invention achieves success in biometric reading with images greater than 1500 DPI.

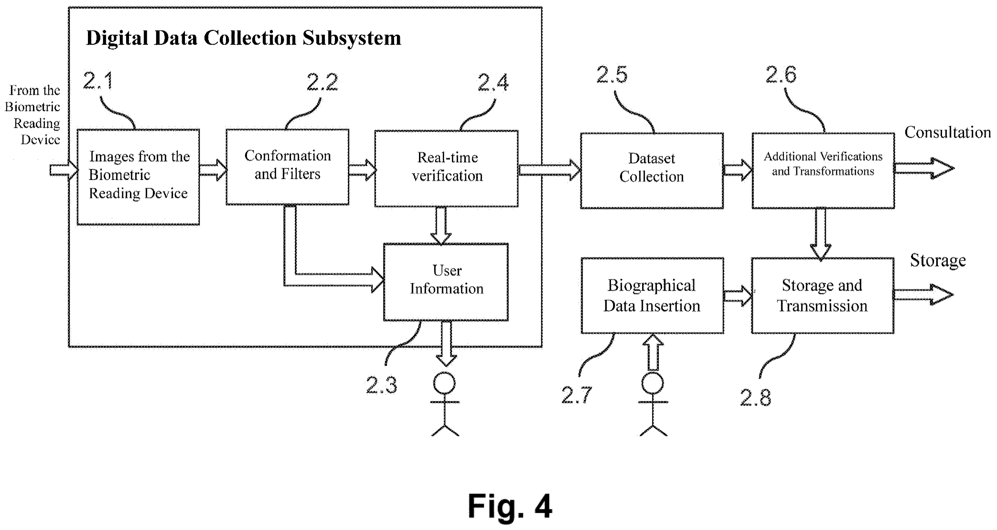

The images captured by this biometric reading device (1.1) need to be processed for collection with the minimum characteristics necessary for the registration or verification process. One challenge to be overcome is that the child may not cooperate with the collection process if he or she is agitated. To minimize and enable collection under these conditions, the process is carried out by the collecting system (1.2), the components of which are detailed in the diagram of Figure 4. Once the images have been collected, they are sent to the storage and consulting system (1.3), where they can be accessed by other systems for viewing, consulting, corrections, and/or fingerprint matching, among other services.

The images from the biometric reading device (2.1) (raw images), collected via the biometric reading device (1.1), are processed by a computational processing module present in a subsystem for collecting digital data, which performs functions such as configuring and filtering (2.2), normalizing, and other conventional image processing methods. This allows these images to be better understood (seen) by the person collecting the information from the user (2.3) and also puts them in a format suitable for a more thorough review process in real-time checks (2.4), which also provides some feedback to the user.

Using the process described above, individual images of each finger are captured and accumulated to form a collection of a set of fingerprints (2.5), typically, this will be the five fingers from the left hand and the five fingers from the right hand and may eventually contain palmprints and footprints.

This set of fingerprint images undergoes a series of further checks and conversions (2.6) to validate the quality of these images in a more refined way, the system being calibrated to improve the quality of the information collected, for example, by combining multiple images. **In** exceptional cases, a new capture can be requested if the pre-established conditions necessary for, as example, a registry to reach a minimum level of quality are not met.

Biographical data (2.7) that identifies the individual is added to this validated set, for example, Name, Mother, Father, and Declaration of Live Birth.

This validated set is stored locally in a storage and transmission component (2.8) and, when transmission is available, it is sent to a storage and consulting system (1.3).

The components of the storage and consulting system (1.3) are detailed in the diagram of Figure 5 that depicts the main functional interfaces of the storage and consulting system (1.3) in a simplified way, comprising an interface for storing (3.1), an interface for consulting (3.2), and an interface for viewing and management (3.3). Its main purpose is to serve as a repository for storing, maintaining, and consulting the images and information collected.

The interface for consulting (3.2) and the interface for viewing and management (3.3) interact via web services (used to transfer data through communication protocols to different platforms, regardless of the programming languages used on those platforms) to enable storage and, if necessary, comparisons for fingerprint matching.

**In** addition, it can operate with these same web services by integrating them with other consulting and collecting systems.

A web page component (3.4) is also provided to allow direct viewing and management of the system by authorized users.

**In** particular, the step performed by the configuring and filtering component (2.2) is detailed in the diagram of Figure 6, in which the raw images collected from the biometric reading device (scanner) vary significantly in terms of position, intensity and filled area. Each collected frame undergoes a processing step that crops the area of interest in the image and performs conversions to normalize the image intensity for subsequent stages in the system.

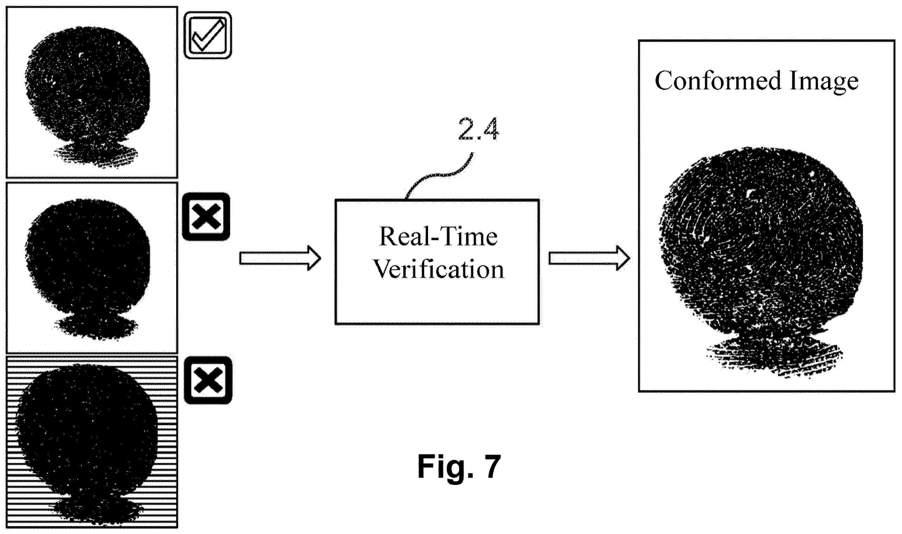

The component that performs the real-time check (2.4) step is detailed in the diagram of Figure 7, in which the previously configured images undergo analysis through a neural network that was previously trained using a set of images collected when this step was not in place (initially annotated by people, and then in a semi-assisted way using previous results). These images are annotated according to their quality, and, on top of this data, a set of neural networks is trained and optimized to be used (generate inferences about the captured image) at the same time as the captures are made. The scores evaluated are based on the classes of valleys, dirt and minutiae trained to segment the image. These networks are also trained through a process of manual annotation.

This process ensures that the best images of each fingerprint are selected with much greater accuracy, guaranteeing their usefulness for future authentication processes.

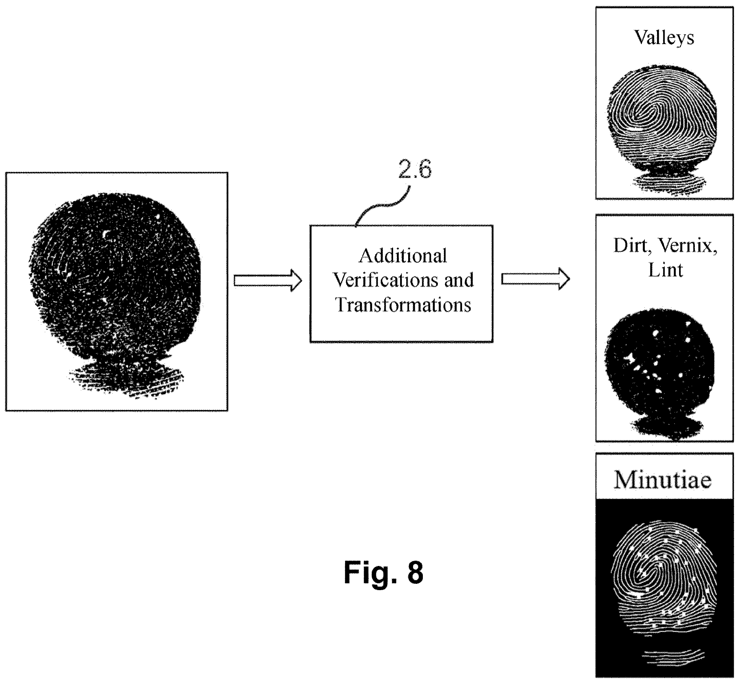

Finally, in the component that performs the step of additional verifications and transformations (2.6), detailed in the diagram of figure 8, the selected set of images goes through a final verification step which consists in validating with minutiae extractors recognized in the market the quality of the obtained images. Through parameterization it is still possible to apply a process of reconstruction of the images from the combination of frames (aligning the images and passing through a trained network to combine and reconstruct the images).

As previously stated, it is also possible that the minimum conditions for collecting have not been met at this checking stage; in this case, the operator is informed which fingerprints need to be collected again or justify the impossibility.

**In** the process of collecting images and identifying children and newborns of the present invention, one problem that needs to be addressed is the uncertainty surrounding differences in image size. **In** a 1:1 matching setting, when the image collected (which must be authentic) matches another previously collected image in the database, the method adopted is to apply scale conversions to the collected image until the distances between the fingerprint valleys are compatible.

An alternative method is to simply apply a set of scale conversions and accept the one that produces the best comparison score ('score' is the metric that numerically assesses whether both images are of the same individual).

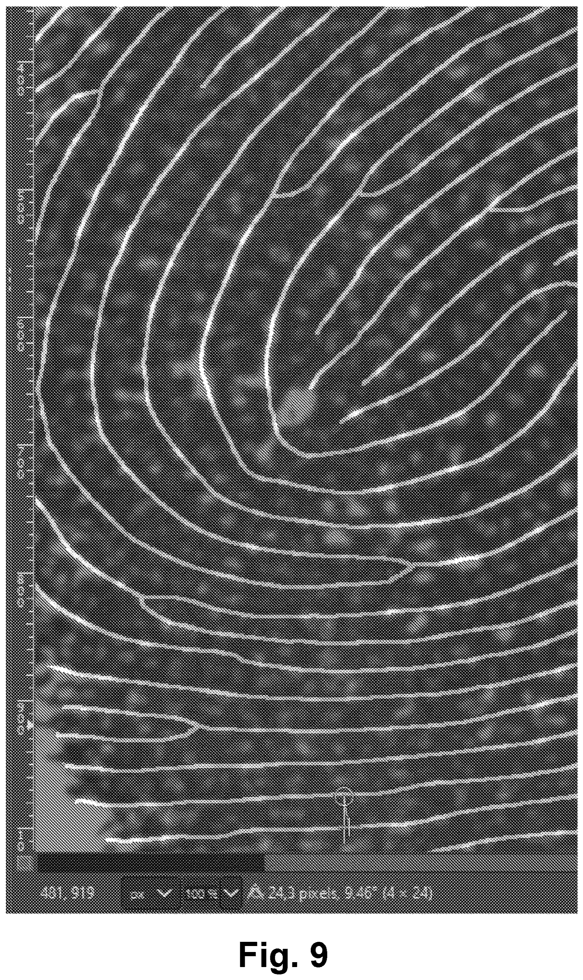

For matchings with a larger number of images (1:N), the base is adjusted so that the templates correspond to the same normalized distance between valleys. As can be seen in Figure 9, the distance between the valleys that can be normalized to create an age-invariant template is indicated.

To describe how the neural network extracts features from fingerprint images of children and newborns, this text presents a description of the methods used to create the neural network for valley segmentation that is used in the solution.

As already described in the step of verifications and additional transformations (2.6), from the input (fingerprint image) are produced the images of valleys among others, the information on valleys being fundamental to biographical identification, as it enables the original image to be processed by state-of-the-art computer tools for extracting fingerprint information, such as Bozorth and Verifinger. It is important to emphasize that, if the original image were to be used without the herein proposed treatment (extraction of the valleys), these tools would indicate an error to inform that the image would not be able to be processed.

In the construction and development of the neural network for extracting characteristics from fingerprint images of children and newborns, the first step is to obtain a dataset of a few hundred high-quality fingerprint images of children, after a process to select the images with the best visual quality, for example: with minimal blur and dirt. Then, experiments are carried out using a reduced dataset to test network architectures that can learn (converge) to the problem in question (i.e., extract the valleys from the original images) and, among the various existing segmentation network architectures, we tested some and achieved good convergence (capacity of the network to learn) using a neural network based on the U-NET architecture.

From the annotation of a single image, it is possible to see that network architectures can learn to generate outputs similar to the annotated image, this network is trained using a loss function known as Dice Loss, which balances false positives (points that should be connected in the image but are not) and false positives (points that should not be connected but are). Dice loss is a loss function used in image segmentation issues to assess the similarity between the predicted and the actual segmentation masks, being used in image segmentation tasks and is a robust measure of similarity that takes into consideration both the precision and the sensitivity of the model.

By way of example and not limitation, the input size of the network used in the present invention is 1280x1280 points, initial attempts with smaller inputs, such as 160x160, produced inferior results, as the network could not 'visualize' a large area. Maintaining the network through a fine resolution somewhat compensates for these artefacts.

The 1280x1280 size strikes a good balance between the required computational resources (memory and CPU) and execution time. Additionally, most newborn and child fingerprints fit within this range, and larger fingerprints can typically be scaled down without a significant loss of accuracy.

Once the model has been selected and the possibility of convergence checked, the supervised learning cycle begins, whose basic principle is to start with a small set of images (initially in the order of dozens) and progressively increase using the models trained throughout the process to generate predictions that will be reviewed (by people who annotate the images using an image editor, such as Photoshop, typically using the model's predictions), this process being repeated until the quality of the predictions is sufficient for them to be put into production. In theory, this cycle could be repeated several times; in the case of the present invention, it took approximately two years to refine with manual annotation of around 500 images. Additionally, images without the aforementioned careful review are used in the training process (via predictions exclusively).

Another relevant technique that allows work with less data is the data augmentation technique, which basically involves converting the input image and, if applicable, the annotation as well. A few of the techniques used include rotations, translations, cropping, scaling, contrasting, and blurring, among others, which also result in a network with more robust results and a lower risk of overfitting.

**In** summary, according to the present invention, enumerating the semi-supervised learning process for segmentation of children's fingerprint images for valleys is made possible in the following steps:
- Data preparation: The first step is to collect and prepare a set of labelled and unlabeled images. The set of labelled images is smaller when compared to the unlabeled set of images.
- Data splitting: The set of images is divided into a labelled training set, an unlabeled training set, and a test set. The labelled training set is used to train the initial model, while the unlabeled training set is used to refine it. The test set is used to evaluate the model's performance.
- Initial model training: The labeled training set is used to train an initial model in a supervised learning algorithm. This model then serves as a starting point for the semi-supervised learning process.
- Prediction generation: The initial model is applied to the unlabeled training set to generate predictions for the unlabeled images.
- Sample selection: Samples with the highest uncertainty in the predictions are selected and labelled manually. These labeled samples are then added to the labelled training set, and the model is retrained.
- Model refinement: The model is refined using the expanded labelled training set and the unlabeled training set. This process can be repeated several times until the model's performance stops improving.
- Model evaluation: After training, the model's performance on unseen data is evaluated using the test set.
- Model application: Finally, the trained model is applied to new images to segment areas of interest and is integrated into the "Collection and Identification System" application according to the present invention.

## Claims

1. "METHOD FOR THE COLLECTION AND IDENTIFICATION OF CHILDREN AND NEWBORNS", **characterized by** comprising the following stages:
Capture images by means of a biometric reading device (1.1) with a resolution greater than 1500 DPI. Collect the images through a collection system (1.2), after which they are sent to a storage system (1.3) being accessible by other systems for viewing, consultation, revision and/or fingerprint confrontation.; Perform the treatment of the images collected by the biometric reading device (2.1) using a computational processing module present in the digital data collection subsystem. This module performs the functions of conforming and filtering (22), normalizing and processing the images; in which the said step performed by the conformation and filtering component (2.2), images are collected by the biometric reading device. Each collected frame undergoes a processing step that identifies the area of interest in the image and performs transformations to normalize the image intensities; Provide visualization of the images by collecting information from the user (2.3) and performing a real-time verification process (2.4). In this process, previously verified images are analyzed using a neural network that has been trained using a set of collected images that were initially annotated and then semi-assisted using previous results. These images are annotated according to their quality, and, on top of this data, a set of neural networks is trained and optimized to operate simultaneously with the captures being made. The scores are then evaluated based on the classes of valleys, dirt and trained minutiae that have been segmented; Capture individual images of each finger, accumulating them to form a collection of fingers (2.5). This collection will typically contain the five fingers of each hand, as well as the palms and soles; Additional checks and transformations (2.6) are performed on the set of finger images to validate their quality. The system is calibrated to improve the quality of the information collected only, and to request a new image capture if the pre-established conditions are not met, in which the selected set of images is verified using recognized minutiae extractors on the market. The images are then reconstructed using a parameterization process involving the combination of frames, image alignment and a trained network for image combination and reconstruction; Insert biographical data (2.7), such as name, mother, father and certificate of live birth, into the validated image set; Store the validated image set locally in a storage and transmission component (2.8) and, when transmission is available, send it to the storage and query system (1.3). This system acts as a repository for storing, maintaining and querying the collected images and information, and provides access via storage (3.1), query (3.2) and viewing and administration (3.3) interfaces; Interact with the consultation interface (3.2) and the visualization and administration interface (3.3) via web services to enable storage and comparison of fingerprints and operate these same web services by integrating them with other query and collection systems; and make the system available via a web page component (3.4) to allow authorized users to view and administer the system directly.

2. "METHOD FOR THE COLLECTION AND IDENTIFICATION OF CHILDREN AND NEWBORNS", According to claim 1, **characterized in that** it also includes a semi-supervised learning process for segmenting images of fingerprints from children and newborns, comprising the following steps:
- Data preparation where the first step is to collect and prepare a set of images, both labeled and unlabeled. The set of labeled images is small compared to the set of unlabeled images.;
- Data splitting where the image set is divided into a labelled training set, an unlabeled training set and a test set. The labelled training set is used to train an initial model, the unlabeled training set is used to refine it, and the test set is used to evaluate its performance;
- Initial model training where a supervised learning algorithm is used to train an initial model on a labelled training set, which is then used as a starting point for the semi-supervised learning process;
- Prediction generation where the initial model is applied to the unlabeled training set to generate predictions for the unlabeled images;
- Sample selection where the samples with the greatest uncertainty in their predictions are manually selected and labeled. These labeled samples are then added to the training set and the model retrained;
- Model refinement where the model is refined using an expanded labeled and unlabeled training set. The refinement process is repeated several times until the model's performance stops improving;
- Model evaluation where, after training, the model is evaluated using the test set to assess its performance on unseen data; and
- Application of the model where the trained model is finally applied to new images to segment the areas of interest and is integrated with the system for the collection and identification of children and newborns.

3. "SYSTEM FOR THE COLLECTION AND IDENTIFICATION OF CHILDREN AND NEWBORNS", **characterized in that** it comprises:
A biometric reading device (1.1) to capture images greater than 1500DP1•,
An image collection system (1.2) comprising a digital image collection subsystem, a component for collecting a set of individual images of each finger (2.5), a component for performing additional checks and transformations (2.6) on the set of finger images and validating their quality, a biographical data insertion component (2.7) for adding information such as name, mother, father and live birth certificate to the validated image set, and a storage and transmission component (2.8) for the validated image set; in which this digital image collection subsystem comprises a component for processing images collected by the biometric reading device (2.1), a component for forming and filtering (2.2), normalization and other classical image processing methods, a component for making images and information available to the user (2.3), and a component for real-time verification (2.4) of previously formed images analyzed by a neural network trained using a set of collected images; and a storage and query system (1.3) that acts as a repository for storing, maintaining and querying the collected images and information comprising a storage interface (3.1) and a query interface (3.2), which interact with an interface for viewing and administration (3.3) via web services. There is also a web page component (3.4) that allows authorized users to view and administer the system directly.
